# EUROPEAN PATENT SPECIFICATION

(11) **EP 0 348 353 B1**
(45) Date of publication and mention of the grant of the patent: **08.09.1993**
(21) Application number: 89810464.1
(22) Date of filing: 16.06.1989
(51) Int. Cl.: A61K 35/12

(54) **The use of physiologically active substances for the manufacture of drugs for cerebral and neuronal diseases**
Verwendung von physiologisch wirksamen Substanzen zur Herstellung von Medikamenten für Gehirn- und Nervenerkrankungen
Application de substances physiologiquement actives pour la fabrication de médicaments pour les maladies cérébrales et nerveuses

(30) Priority: 20.06.1988 JP 152492/88
(43) Date of publication of application: 27.12.1989
(73) Proprietor: NIPPON ZOKI PHARMACEUTICAL CO. LTD., Osaka (JP)
(72) Inventor: Konishi, Jin-emon, c/o Inst. of Bio-Active Science, Yashiro-cho, Katoh-gun, Hyogo (JP)
(74) Representative: Kerr, Andrew

(56) References cited:
- BIOLOGICAL ABSTRACTS, vol. 63, no. 7, 1977, Biological Abstracts Inc., Philadelphia, PA (US); T. KITA et al., no. 41007#
- BIOLOGICAL ABSTRACTS, vol. 85, no. 8, 1988, Biological Abstracts Inc., Philadelphia, PA (US); C. HARA et al., no. 82541#
- BIOLOGICAL ABSTRACTS, vol. 87, no. 4, 1989, Biological Abstracts Inc., Philadelphia, PA (US); T. HATA et al., no. 41156#
- BIOLOGICAL ABSTRACTS, vol. 87, no. 11, 1989, Biological Abstracts Inc., Philadelphia, PA (US); M. TANAKA et al., no. 117949#
- BIOCHEM. J., vol. 34, 1940; R.J.L. ALLEN, pp. 858-865#
- ANALYTICAL CHEMISTRY I, 2nd revised ed., Y. Tanaka & Y. Ohkuru (editors), Nenzando Co.#
- HANDBOOK OF ANALYTICAL CHEMISTRY, 3rd revised ed., Japanese Society for Analytical Chemistry, Maruzen Co.#

## Description

The present invention relates to the use of physiologically active substances extracted from infected tissues for the manufacture of drugs for cerebral and neuronal diseases.

Nerve Growth Factor (NGF) has biological activities stimulating the differentiation and growth of sympathetic and sensory neurons. It has been proposed that there is a relationship between NGF and neuronal diseases affecting peripheral nerves and for this reason NGF has been proposed for the treatment of diseases of this type. In addition, it has recently been found that NGF is produced in the brain and that it has physiological activity there. A correlation has been shown between NGF and the cause of Alzheimer's senile dementia and the use of NGF for the treatment of dementia has been proposed accordingly. Since NGF is a proteinic factor, a number of factors, such as route of administration and pharmaceutical dosage form must, however, be clarified before NGF may be administered to humans. There is therefore a need to develop drugs having a nerve growth stimulating effect similar to that of NGF, but which can easily be administered to humans and which provide greater safety without any undesirable side effects.

A substance designated Neurotropin (NSP) is described in Biol. Abstr. 63:41007, 85:82541, 87:41156 and 87:117949. It is said to be an extract containing many conjugated polysaccharides isolated from the skin or tissues of rabbits which have been inoculated with living cowpox virus. It is described as having analgesic, immunopotentiation, and anti-ulcerogenic properties and to affect noradrenaline metabolism in tests on rats and mice. Neither the composition nor extraction process have been published.

It has now been found that physiologically active substances extracted from infected tissues and produced by inoculation with a poxvirus to animal tissues, organs or cultured cells exert an excellent NGF-like action on the nerve system.

The present invention provides for the use of a physiölogically active substance for the manufacture of drugs for cerebral and neuronal diseases, wherein the substance is obtainable by a process characterised by the following steps:-
i. tissues, organs or cultured cells obtained from an animal infected with a poxvirus are homogenised with an aqueous extraction medium,
ii. the resulting extract is subjected to deproteinization,
iii. the deproteinized extract is adsorbed on an adsorbent,
iv. the adsorbed material is eluted with a basic aqueous elutant solution.

Term "infected tissues" as used in this specification is defined as meaning: animal tissues, organs or cultured cells inoculated or otherwise infected with a poxvirus.

The preferred process and resulting product are hereafter described.

A poxvirus, for example orthopox virus such as vaccinia virus, cowpox virus, variola virus, infectious ectromelia virus or monkey pox virus; parapoxvirus such as orf virus, paravaccinia virus or bovine papular stomatitis virus; capricopoxvirus such as sheeppox virus, goatpox virus or lumpy skin diseases virus; avipoxvirus such as fowlpox virus or hare fibroma virus, leporipoxvirus such as rabbit myxoma virus or rabbit fibromas virus, swinepoxvirus, Yaba monkey tumor virus or Tarapox virus, can be used.

The infected tissues may be collected from various kinds of animals or birds, such as rabbit, sheep, goat, pig, cow, horse, monkey, hamster, guinea pig, rat, mouse or hen. The animal or bird may be selected according to a species of poxvirus and to other conditions. Any type of cultured cell in which the selected poxvirus can multiply may also be selected, such as cultured cells or tumour cells of kidney, skin, lung, testis, liver, muscle, adrenal or thyroid gland, brain, nerve cell or blood cell of rabbit, sheep, goat, pig, cow, horse, monkey, hamster, guinea pig, rat, mouse, hen or embryos thereof. Cultured cells derived from humans, such as Hela cell or decidua of the hatching egg may also be employed.

The infected tissues are collected under aseptic conditions and ground until they are as small as possible. After addition of an extraction medium to the ground material the product is then homogenized. Extraction media that may be used include distilled water, physiological saline, weakly acidic or basic buffers etc. If desired a stabilizer such as glycerin, a disinfetant or preservative such as phenol, or an inorganic salt such as sodium chloride, potassium chloride or magnesium chloride may be added to the medium. Extraction may be facilitated by a procedure designed to disintegrate cell tissues, such as freeze-thaw extraction, sonication or treatment with a detergent or an enzyme which dissolves cell membrane.

The resulting emulsion is filtered or centrifuged to remove tissue fragments. The filtrate or supernatant is deproteinized according to a known method, for example by heating, sonication, treatment with a protein denaturant such as an acid, a base, urea, guanidine, an organic solvent or a detergent, iso-electric point precipitation or salting-out technique. The denatured proteins precipitated in this manner are then removed by filtration using a filter paper made of, for example, cellulose or nitrocellulose, a glass filter, Sellaite®, Seitz's filter, etc., ultrafiltration, gel filtration, ion-exchange chromatography or centrifugation.

The resulting extract containing the active substances is acidified, preferably to a pH of 3.5 - 5.5 by addition of an acid such as hydrochloric acid, sulfuric acid or hydrobromic acid, and then subjected to adsorption on an adsorbent such as active carbon, kaolin or an ion-exchange resin. The adsorbent may be added to the extracted solution with subsequent stirring, or alternatively the extracted solution may be passed through a column containing the adsorbent.

To elute the material containing the active substances of the present invention a basic aqueous solution is added to the adsorbent, the suspension is preferably adjusted to a pH of from 9 to 12 and the mixture is then incubated or stirred at room temperature or heated to a suitable temperature above room temperature. Elution is effected by removing the adsorbent according to a known method, such as filtration or centrifugation. The eluate thus obtained is preferably adjusted to a pH of 6.5 - 8.5 and then concentrated to dryness under reduced pressure or, alternatively, lyophilized to yield the active substances of the invention.

The physical and chemical properties of the physiologically active substances obtained as set out above are as follows:
1) Appearance: Pale yellowish brown, hygroscopic powder.
2) Solubility Soluble in water, methanol and ethanol.
3) Ultraviolet adsorption: λmax = 255 - 275 nm.
4) Ninhydrin reaction: positive.
5) One ml of perchloric acid is added to 2 mg of the substances of the present invention and the mixture heated until the solution becomes colourless. 3 ml of dilute hydrochloric acid, 0.4 g of amidol hydrochloride and 8 g of sodium hydrogen sulfite are dissolved in 100 ml of water. 2 ml of the resulting aqueous solution are then mixed with 1 g of ammonium molybdate and 30 ml of water. 2 ml of the resulting mixture is then added to the above solution which contains the substances of the present invention. The resulting solution is blue in colour, indicating the presence of phosphorus.
6) 5 mg of the above solution of the present invention are dissolved in 10 ml of water. 0.2 g of orcine and 0.135 g of iron(II)ammonium sulfate are dissolved in 5 ml of ethanol, 83 ml of hydrochloric acid are added to the mixture which is then made up to 100 ml through addition of water. 3 ml of the resultant mixture are added to 1 ml of the above solution containing the substances of the invention and heated on a water bath. The solution obtained is green in colour, indicating the presence of pentose.
7) Silver nitrate reagent is added to the aqueous solution of the substances of the present invention and a precipitate is formed, indicating the presence of chloride.
8) The resulting precipitate contains bases of nucleic acids.
9) Various protein detection methods carried out in the resulting precipitate yielded negative results.

The following examples, which serve merely as illustration and are not intended in any way to limit the scope of the invention, describe the preparation of the physiologically active substances of the present invention.

### Example 1

Vaccinia virus was inoculated into the skin of a healthy adult rabbit. The inflamed skin was cut off under aseptic conditions and well macerated. Aqueous phenol solution was added to the ground material, homogenized and the emulsion filtered by centrifugation.The resulting filtrate was adjusted to a pH of from 4.5 to 5.5 and then heated in a stream of 100°C steam. The proteins precipitated in this procedure were removed by filtration, the filtrate was adjusted to a pH of 8.5 to 10.00 by addition of sodium hydroxide, heated to 100°C and filtered. The filtrate was adjusted to a pH of 4.5 and 1.5% active carbon was added thereto. The suspension was stirred for 1 to 5 hours and then filtered. Water was added to the resulting active carbon and the suspension was adjusted to a pH of from 9.4 to 10.0 through addition of sodium hydroxide. The extraction procedure was carried out by stirring for 3 - 5 hours at 60°C. The suspension was filtered to remove the active carbon. The filtrate was adjusted to a near neutral pH (ca. pH 7) by addition of hydrochloric acid and concentrated to dryness under reduced pressure to yield the substances of the present invention. The yield of the substances of the present invention was 1.5 - 2.0 g per 1 kg of infected skin tissues.

### Example 2

Methanol was added to the basic aqueous suspension of active carbon having absorbed thereon the deproteinised extract obtained in the same manner as in Example 1 and stirred for 1 hour. The mixture was filtered and the filtrate concentrated to dryness under reduced pressure to yield the substances of the present invention. The yield was 4.0 - 6.0 g per 1 kg of infected skin tissues.

The following descriptions serve to illustrate pharmaceutical studies of the substances of the present invention.

### (1) Toxicity test

In an acute toxicity test, the physiologically active substances of the present invention were administered to male and female mice and rats orally, subcutaneously, intraperitoneally and intravenously. The LD₅₀ of the substances of the invention was more than 5,000 mg/kg for any of the routes of administration, regardless of the species and sex of the animals used.

Subacute toxicity tests revealed no abnormality in any of the organs. Reproduction tests revealed no effects on pregnant animal, foetus, newborn or reproductivity of the offspring (F₁).

### (2) Nerve growth stimulating activity

The nerve growth stimulating activity of the physiologically active substances of the present invention was investigated using PC12h cells (a rat pheochromocytoma cell line) which respond to NGF by differentiating into sympathetic neuron-like cells.

PC12h cells were plated on a collagen-coated 24-well microplate in DF medium containing 5% horse serum and 5% precolostrum newborn calf serum. After culturing overnight, the medium was replaced with a serum-free medium (DF medium supplemented with human transferrin, bovine insulin and progesterone) with 100 ug/ml of the substances of the present invention and incubated for 3 days under 90% air and 10% CO₂ gas at 37^{o}C. The rate of the number of cell with neurite processes (≧20µm) was determined by counting cells at random in 10 fields under a phase contrast microscope.

The substances of the present invention were found to have NGF-like neurotrophic activity in respect of neurite outgrowth and cell surface change.

### (3) Clinical study

Pharmaceutical compositions containing as active ingredient the substances of the present invention were administered to patients suffering from post-herpetic neuralgia, brain edema, dementia and spino-cerebellar degeneration.

### I. Post-herpetic neuralgia

8 mg of the substances of the present invention were administered orally twice daily to patients suffering from post-herpetic neuralgia for a period of 4 weeks. More than 63% of patients showed more than slight improvement, moderate to marked improvement being noted in about 50% of patients.

### II. Brain edema

10 mg to 36 mg of the substances of the invention were administered daily intravenously or by instillation to patients suffering from brain edema for one to two weeks. Treatment was then contined, depending on the condition of the patient, by administering a daily dosage of 8 mg to 16 mg of the substance of the invention orally for a few weeks.

The efficacy of the treatment was monitored by a time course comparison of edema size using a CT scan and by observing symptoms, using a neurological evaluation rating scale.

Comparing the efficacy of the substances of the present invention with that of steroid hormone therapy, the results on the 10th day of treatment showed that the substances of the invention were significantly superior to those produced by steroid hormones. The substances of the invention produced an improvement in 63% of patients, only 42% of patients showing an improvement after steroid hormone therapy.

### III. Dementia

Clinical studies were carried out on patients suffering from cerebral organic disorders, vascular dementia and Alzheimer's dementia. About 10 mg of the substances of the present invention were administered daily to these patients intravenously or by instillation.

Efficacy was evaluated by Hasegawa's simple mental function evaluation scale, slcoring degrees of psychopathological conditions such as volition and emotion, scoring degrees of actions in daily life (ADL), clinical tests such as EEG and CT scan and GBS scale (a rating scale for dementia syndromes).

Of the patients treated for 8 weeks with the substances of the present invention, more than about 70% showed improvements in motility, volition, speech disorders, attention, memory, incontinence and emotional disorders.

### IV. Spino-cerebellar degeneration

3 mg to 8 mg of the substances of the present invention were administered daily intravenously to 4 members of a family suffering from spino-cerebellar degeneration. 2 patients were in a terminal stage and failed to respond to treatment. Of the 2 remaining cases, were the disease was evolutive, a spectacular improvement was observed in such symptoms as incontinence, motor incoordination, defective vision, nocturnal spasm and ataxic gait. The effect became evident after about 2 months of treatment.

In one evolutive case, treatment was discontinued for 2 weeks. All the pathological symptoms that reappeared during this period disappeared again when treatment with the substances of the present invention was resumed.

No severe side effects were reported during these clinical trials, only such side effects a sleeplessness, sweating, thirst and gastrointestinal disorders being reported.

It follows from the above-mentioned results that the physiologically active substances of the present invention have an NGF-like nerve growth stimulating effect and can consequently repair injured cerebral and nerve cells or cells having diminished physiological function. It follows that the substances of the invention are useful as drugs for the treatment of vascular dementia caused by cerebral arteriosclerosis, postencephalitis, postapopleptic disorder or post-traumatic syndrome after head injury, Alzheimer's disease including Alzheimer's senile dementia, subcortical dementia such as Huntington's chorea or Parkinson's disease, cerebral diseases such as brain edema and spinocerebellar degeneration, and neuronal diseases such as autonomic imbalance and post-herpetic neuralgia caused by morbidity or injury of sympathetic nerves or sensory nerves.

The substances of the invention have low toxicity and great safety. They are consequently suitable for oral long-term, continuous administration.

The substances of the present invention may be made up into pharmaceutical compositions through combination with appropriate pharmaceutically acceptable carries or diluents, and may be formulated into preparations in solid, semisolid, liquid or gaseous form such as tablets, capsules, powders, granules, solutions and suppositories in a conventional manner for oral or parenteral administration.

In pharmaceutical dosage forms, the substances of the present invention may be used alone or in appropriate association, as well as in combination with other pharmaceutically active components.

In the case of oral preparations, the substances may be used alone or in combination with appropriate adjuvants in the form of tablets, powders, granules or capsules, e.g. with conventional adjuvants such as lactose, mannitol, corn starch or potato starch; with binders such as crystalline cellulose, cellulose derivatives, acacia, corn starch or gelatins; with disintegrators such as corn starch, potato starch or calcium carboxymethylcellulose; with lubricants such as talc or magnesium stearate; and if desired, with diluents, buffering agents, moistening agents, preservatives and flavoring agents.

The substances of the present invention may be formulated into preparations for injection by dissolving, suspending or emulsifying in aqueous or non-aqueous solvents, such as distilled water for injection purposes, physiological saline solution, 5 - 20% glucose aqueous solution, vegetable oil, synthetic aliphatic acid glycerides, esters of higher aliphatic acid or propylene glycol; and if desired, with conventional adjuvants such as solubilizers, isotonic agents, suspending agents, emulsifying agents, stabilizers and preservatives. The substances of the present invention may also be formulated into lyophilized preparations in vials, which are used as injections by dissolving with the said solvent.

Furthermore, the substances of the invention may be made up into suppositories by mixing with a variety of bases, e.g. emulsifying bases or water-soluble bases, and may also be made up into inhalations or aerosol preparations.

The preferred dose of the substances of the present invention varies with the subject, the form of the drug, the method and period of administration. However, in order to obtain useful effects, it is generally recommended to administer 1 to 100 mg orally, preferably 4 to 40 mg daily.

In the case of parenteral administration, e.g. injections, doses of the substance in the order of one tenth to one third of the above dose are preferable as daily doses.

Pharmaceutical compositions which contain the substances of the present invention as an active ingredient are described below.

| Formulation Example 1 (Tablet) | |
|---|---|
| Ingredient | Amount in one tablet (mg) |
| Substance of the invention | 4 |
| Lactose | 106 |
| Crystalline cellulose | 40 |
| Calcium carboxymethylcellulose | 20 |
| Magnesium stearate | 10 |
| | Total 180 mg |

| Formulation Example 2 (Capsule) | |
|---|---|
| Ingredient | Amount in one capsule (mg) |
| Substance of the invention | 10 |
| Lactose | 200 |
| Talc | 40 |
| | Total 250 mg |

| Formulation Example 3 (Injection) | |
|---|---|
| Ingredient | Amount in one ampoule (mg) |
| Substance of the invention | 1 |
| Sodium chloride | appropriate amount |
| Distilled water for injection | appropriate amount |
| | Total 1 ml |

## Claims

1. The use of a physiologically active substance for the manufacture of drugs for cerebral and neuronal diseases, wherein the substance is obtainable by a process characterized by the following steps:-
i. tissue, organs or cultured cells obtained from an animal infected with a poxvirus are homogenised with an aqueous extraction medium,
ii. the resulting extract is subjected to deproteinization,
iii. the deproteinized extract is adsorbed on an adsorbent,
iv. the adsorbed material is eluted with a basic aqueous elutant solution.

2. The use as claimed in claim 1, wherein the substance possesses:
1) Appearance: pale yellowish brown and hygroscopic powder.
2) Solubility: Soluble in water, methanol and ethanol.
3) Ultraviolet adsorption: λmax = 255-275 nm.
4) Ninhydrin reaction: Positive
5) Qualitative analysis of phosphorus: Positive.
6) Qualitative analysis of pentose: Positive.
7) Qualitative analysis of chloride ion: Positive.
8) Contains bases of nucleic acids.
9) Various methods of protein detection are negative.

3. The use as claimed in the claims 1 or 2 for the manufacture of drugs for the treatment of dementia.

4. The use as claimed in claims 1 or 2 for the manufacture of drugs for the treatment of cerebral diseases.

5. The use as claimed in claim 4 for the manufacture of drugs for the treatment of brain edema.

6. The use as claimed in claim 4 for the manufacture of drugs for the treatment of spino-cerebellar degeneration.

7. The use as claimed in claim 1 or 2 for the manufacture of drugs for the treatment of neuronal diseases.

8. The use as claimed in claim 7 for the manufacture of drugs for the treatment of post-herpetic neuralgia.

9. The use as claimed in any one of the claims 1 to 8, wherein drugs are formulated into a form suitable for oral administration.

10. The use as claimed in any one of the claims 1 to 8, wherein drugs are formulated into a form suitable for parenteral administration.

## Patentansprüche

1. Verwendung einer physiologisch wirksamen Substanz zur Herstellung eines Medikaments für Hirn- und Nervenerkrankungen, worin die Substanz durch ein Verfahren erhältlich ist, das durch die folgenden Schritte gekennzeichnet ist:-
i. aus einem mit einem Pockenvirus infizierten Tier erhaltene Gewebe, Organe oder Kulturzellen werden mit einem wässrigen Extraktionsmedium homogenisiert,
ii. der resultierende Extrakt wird Deproteinisierung unterworfen,
iii. der deproteinisierte Extrakt wird an ein Adsorbens adsorbiert,
iv. das adsorbierte Material wird mit einer basischen wässrigen Eluierungslösung eluiert.

2. Verwendung wie in Anspruch 1 beansprucht, worin die Substanz folgende Eigenschaften besitzt:
1) Aussehen: blassgelblichbraunes und hygroskopisches Pulver.
2) Löslichkeit: löslich in Wasser, Methanol und Äthanol.
3) Ultraviolettabsorption: λmax = 255-275 nm.
4) Ninhydrinreaktion: positiv
5) Qualitative Phosphoranalyse: Positiv.
6) Qualitative Pentoseanalyse: Positiv.
7) Qualitative Chlorionenanalyse: Positiv.
8) Enthält Basen der Nukleinsäuren.
9) Verschiedene Proteinnachweisverfahren sind negativ.

3. Verwendung wie in Ansprüchen 1 oder 2 beansprucht, zur Herstellung von Medikamenten für die Behandlung von Dementia.

4. Verwendung wie in Ansprüchen 1 oder 2 beansprucht, zur Herstellung von Medikamenten für die Behandlung von Hirnerkrankungen.

5. Verwendung wie in Anspruch 4 beansprucht, zur Herstellung von Medikamenten für die Behandlung von Hirnödem.

6. Verwendung wie in Anspruch 4 beansprucht, zur Herstellung von Medikamenten für die Behandlung von Rückenmarkdegeneration.

7. Verwendung wie in Anspruch 1 oder 2 beansprucht, zur Herstellung von Medikamenten für die Behandlung von Nervenerkrankungen.

8. Verwendung wie in Anspruch 7 beansprucht, zur Herstellung von Medikamenten für die Behandlung von postherpetischer Neuralgie.

9. Verwendung wie in irgendeinem der Ansprüche 1 bis 8 beansprucht, worin die Medikamente in eine Form, die für orale Verabreichung geeignet ist, formuliert werden.

10. Verwendung wie in irgendeinem der Ansprüche 1 bis 8 beansprucht, worin die Medikamente in eine Form, die für parenterale Verabreichung geeignet ist, formuliert werden.

## Revendications

1. L'utilisation d'une substance physiologiquement active pour la fabrication de médicaments pour traiter des maladies cérébrales et neuronales, utilisation dans laquelle la substance peut être obtenue par un processus caractérisé par les étapes suivantes :
i) homogénéisation d'un tissu, d'organes, ou de cellules de culture obtenus à partir d'un animal infecté par un poxviridé, au moyen d'un milieu d'extraction aqueux,
ii) déprotéinisation de l'extrait résultant,
iii) adsorption de l'extrait déprotéinisé sur un adsorbant, et
iv) élution de la matière adsorbée par une solution éluante aqueuse basique.

2. L'utilisation de la revendication 1, dans laquelle la substance possède les caractéristiques suivantes :
1) Aspect : poudre brun-jaunâtre pâle et hygroscopique.
2) Solubilité : soluble dans l'eau, dans le méthanol et dans l'éthanol.
3) Adsorption ultraviolette : λₘₐₓ = 255-275 nm.
4) Réaction à la ninhydrine : positive.
5) Analyse qualitative du phosphore : positive.
6) Analyse qualitative des pentoses : positive.
7) Analyse qualitative de l'ion chlorure : positive.
8) Contient des bases d'acide nucléique.
9) Les divers procédés de détection des protéines sont négatifs.

3. L'utilisation de la revendication 1 ou 2, pour la fabrication de médicaments pour le traitement des démences.

4. L'utilisation de la revendication 1 ou 2, pour la fabrication de médicaments pour le traitement de maladies cérébrales.

5. L'utilisation de la revendication 4, pour la fabrication de médicaments pour le traitement de l'oedème du cerveau.

6. L'utilisation de la revendication 4, pour la fabrication de médicaments pour le traitement des dégénérescences spino-cérébellaires.

7. L'utilisation de la revendication 1 ou 2, pour la fabrication de médicaments pour le traitement de maladies neuronales.

8. L'utilisation de la revendication 7, pour la fabrication de médicaments pour le traitement des névralgies post-herpétiques.

9. L'utilisation de l'une quelconque des revendications 1 à 8, dans laquelle les médicaments sont formulés sous une forme appropriée à une administration orale.

10. L'utilisation de l'une quelconque des revendications 1 à 8, dans laquelle les médicaments sont formulés en une forme appropriée à une administration parentérale.
